Europäisches Patentamt

European Patent Office

Office européen des brevets

(19)

(11) Veröffentlichungsnummer: **0 320 840**

**A2**

# (12) EUROPÄISCHE PATENTANMELDUNG

(21) Anmeldenummer: 88120699.9

(22) Anmeldetag: 10.12.88

(51) Int. Cl.⁴: **C07K 7/00 , C07K 7/08 , C07K 17/08 , C07K 1/00 , A61K 39/395 , G01N 33/68**

Patentansprüche für folgende Vertragsstaaten:
ES + GR.

(30) Priorität: **18.12.87 DE 3742997**

(43) Veröffentlichungstag der Anmeldung:
**21.06.89 Patentblatt 89/25**

(84) Benannte Vertragsstaaten:
**AT BE CH DE ES FR GB GR IT LI LU NL SE**

(71) Anmelder: **BEHRINGWERKE AKTIENGESELLSCHAFT**
**Postfach 1140**
**D-3550 Marburg/Lahn(DE)**

(72) Erfinder: **Stief, Thomas, Dr.**
**Friedrichstrasse 40**
**D-3550 Marburg(DE)**
Erfinder: **Stüber, Werner, Dr.**
**Cölber Weg 12**
**D-3551 Lahntal(DE)**

(74) Vertreter: **Meyer-Dulheuer, Karl-Hermann, Dr. et al**
**HOECHST Aktiengesellschaft Zentrale Patentabteilung Postfach 80 03 20**
**D-6230 Frankfurt/Main 80(DE)**

(54) **Peptide, Verfahren zu ihrer Herstellung, ihre Verwendung zur Gewinnung von Antikörpern sowie deren Verwendung zur Blockierung der PAI-1-Aktivität menschlichen Blutes.**

(57) Es werden Peptide, enthaltend die Aminosäuresequenz des Peptids Ser-Thr-Ala-Val-Ile-Val-Ser-Ala-Arg-Met-Ala-Pro-Glu-Glu-Ile-Ile oder einem Teil davon, ein Verfahren zu ihrer Herstellung, Antikörper, die durch Immunisierung von Tieren mit jenen Peptiden erzeugt werden, wobei diese Antikörper zur Blockierung von PAI-1 im menschlichen Blut dienen, beschrieben.

EP 0 320 840 A2

## Peptide, Verfahren zu ihrer Herstellung, ihre Verwendung zur Gewinnung von Antikörpern sowie deren Verwendung zur Blockierung der PAI-1-Aktivität menschlichen Blutes

Die Erfindung betrifft Peptide, welche die Aminosäuresequenz des aktiven Zentrums des endotheliären Plasminogenaktivator-Inhibitors (PAI-1) enthalten, ein Verfahren zu ihrer Herstellung, Antikörper, die durch Immunisieren von Tieren mit jenen Peptiden erzeugt werden, sowie die Verwendung dieser Antikörper zur Blockierung von PAI-1 im menschlichen Blut.

Seit kurzem ist bekannt, daß die Aktivität der beiden physiologisch bedeutendsten humanen Plasminogenaktivatoren t-PA und u-PA über zwei spezifische Inhibitoren im Blut reguliert wird. Dabei handelt es sich einerseits um den endotheliären Plasminogenaktivator-Inhibitor, genannt PAI-1, der unter anderem von Endothelzellen und Thrombozyten gebildet und freigesetzt wird, andererseits um den plazentären Plasminogenaktivator-Inhibitor, genannt PAI-2, aus den Trophoblastzellen der Plazenta. Beide Inhibitoren gleichen sich in ihren hohen Assoziationskonstanten mit Plasminogenaktivatoren, sind allerdings immunologisch nicht verwandt.

Klinisch scheint diesen Inhibitoren eine große Bedeutung zuzukommen, da insbesondere stark erhöhte Konzentrationen an PAI-1 zu lebensbedrohlichen Komplikationen führen können. Solche Zusammenhänge sind bereits bei Patienten mit tiefer Beinvenenthrombose, akutem Herzinfarkt, bakteriellem Blutbefall, Lebererkrankung, Bauchspeicheldrüsenentzündung, Krebsleiden sowie in der Spätphase der Schwangerschaft und bei Patientinnen mit Schwangerschaftstoxikose beschrieben worden.

Von diagnostischem wie therapeutischem Interesse ist eine Substanz, die in der Lage ist, die Aktivität dieses Inhibitors spezifisch zu blockieren.

Überraschenderweise wurde gefunden, daß die PAI-1-Aktivität mit Antikörpern blockiert werden kann, die erhalten werden, wenn man beispielsweise ein Peptid der Sequenz Ser-Thr-Ala-Val-Ile-Val-Ser-Ala-Arg-Met-Ala-Pro-Glu-Glu-Ile-Ile, das gegebenenfalls an ein Trägermolekül gekoppelt ist, als Immunogen verwendet.

Gegenstand der Erfindung ist deshalb ein Peptid enthaltend von 4 bis zu 40 Aminosäuren, dessen Sequenz mit der Aminosäuresequenz des Peptids Ser-Thr-Ala-Val-Ile-Val-Ser-Ala-Arg-Met-Ala-Pro-Glu-Glu-Ile-Ile oder mit Teilen dieser Sequenz übereinstimmt.

Von besonderer Bedeutung erscheint die Sequenz -Arg-Met-, so daß Peptide bevorzugt sind, die diese Sequenz enthalten.

Bevorzugt sind Peptide mit 6 bis 25 Aminosäuren, wobei die Arg-Met-Sequenz von wenigstens 2 Aminosäuren flankiert ist.

Besonders bevorzugt sind die Peptide Ser-Thr-Ala-Val-Ile-Val-Ser-Ala-Arg-Met-Ala-Pro-Glu-Glu- Ile-Ile oder Cys-Ser-Thr-Ala-Val-Ile-Val-Ser-Ala-Arg-Met-Ala-Pro-Glu-Glu-Ile-Ile.

Solche Peptide sind zur Gewinnung im Rahmen der Erfindung geeigneter Antikörper brauchbar.

Solche Antikörper können mit Hilfe der entsprechenden Peptide immunadsorptiv gereinigt werden. Diese Antikörper blockieren spezifisch die Aktivität des im menschlichen Blut vorhandenen PAI-1.

Die Herstellung der erfindungsgemäßen Peptide erfolgt nach an sich bekannten Verfahren, beispielsweise nach der klassischen, in Lösung arbeitenden Technik, wobei geschützte Aminosäuren oder Peptidsegmente aneinander kondensiert werden und nach Abspaltung der Schutzgruppen das gewünschte Peptid erhalten wird. Besonders bevorzugt werden die erfindungsgemäßen Peptide jedoch nach der Festphasenmethode hergestellt, so daß ein Verfahren zur Herstellung dieser Peptide ebenfalls Gegenstand der Erfindung ist.

Bei der Festphasenpeptidsynthese werden die Peptide an einer Matrix, wie quervernetztem Polystryrol, Polyacrylamid oder dergleichen, die gegebenenfalls mit Ankermolekülen versehen sind, aufgebaut und davon abgespalten. Vorzugsweise wurde eine unlösliche Polystyrolmatrix (1 % quervernetzt mit Divinylbenzol) zu diesen Synthesen benutzt, die eine Beladung mit der C-terminalen Aminosäure in Höhe von 0.1 - 1 mmol/g, bevorzugt 0.4 - 0.6 mmol/g Harz, aufwies. Da der Aufbau der Peptide unter Benutzung der basenlabilen Fmoc-Gruppierung erfolgte, wurden als Ankermolekül p-Alkoxybenzylesterverbindungen nach dem Stand der Technik benutzt. Als Lösemittel wurden bei der Synthese vorzugsweise Dichlormethan, N-Methylpyrrolidon und ganz besonders bevorzugt Dimethylformamid angewandt. Bei jedem Wasch- oder Reaktionsschritt wurden üblicherweise 10 - 25 ml/g Harz Lösung bzw. Lösemittel eingesetzt, wobei 15 ml besonders bevorzugt wurden. Da die alpha-$NH_2$-Funktionen der Aminosäuren mit der Fmoc-Gruppe geschützt waren, wurde für die trifunktionellen Aminosäuren folgende Schutzgruppenwahl der Seitenfunktionen getroffen:

| Serin, Threonin | tert.-Butylether |
|---|---|
| Glutaminsäure | tert.-Butylester |
| Arginin | 4-Methoxy-2,3,6-trimethylphenylsulfonyl- |
| Cystein | tert.-Butylmercapto. |

Die Aktivierung und Kopplung der Aminosäuren erfolgte beispielsweise über Aktivester, gemischte oder symmetrische Anhydride, wobei jedoch eine in situ-Aktivierung mit HOBt/Carbodiimid, besonders bevorzugt N,N'-Diisopropylcarbodiimid, erfolgte.

Die Abspaltung der alpha-NH$_2$-Schutzgruppen erfolgte mit einer Base, wobei besonders bevorzugt 20 % Piperidin in DMF bei Raumtemperatur eingesetzt wurden. Nach jedem dieser Reaktionsschritte wurde das Harz gewaschen, vorzugsweise mit DMF und Isopropylalkohol.

Die Abspaltung der Peptide vom Träger erfolgte vorzugsweise acidolytisch unter gleichzeitiger Entfernung der Seitenfunktionschutzgruppen. Die Sulfhydrylfunktion des Cysteins wurde durch Thiole, wie Dithiothreitol oder bevorzugt durch Butylphosphin freigesetzt. Die erfindungsgemäßen Peptide wurden nach an sich bekannten Methoden aufgereinigt, beispielsweise durch Gelpermeation an Agarosegelen, bevorzugt jedoch durch Hochleistungsflüssigkeitschromatographie.

Die synthetischen Peptide wurden bezüglich ihrer chemischen Zusammensetzung und Reinheit untersucht. Die Zusammensetzung der Peptide wurde durch Aminosäurenanalyse bestimmt. Zu diesem Zweck wurde eine kleine Probe mit 6 N Salzsäure in Gegenwart von Phenol 24 Stunden bzw. 72 Stunden bei 110° C erhitzt. Damit wurde nachgewiesen, daß die Aminosäuren im Erwartungsbereich in die Peptidkette eingebaut wurden, und ebenso konnte damit der Peptidgehalt der synthetischen Antigene mit gleich oder größer als 85 % bestimmt werden.

Die Reinheit der Peptide wurde durch die Hochleistungsflüssigkeitschromatographie an C18-Umkehrphasen bestimmt. Für diesen Zweck wurde ein Phosphat-/Acetonitrilgradient eingesetzt. Es wurde gezeigt, daß die Peptide eine Reinheit von größer als 85 % hatten.

Zur Gewinnung von Antikörpern wird ein solches Peptid gegebenenfalls über einen Cysteinrest an einen hochmolekularen Träger gebunden. Mit einem solchen Produkt werden Tiere immunisiert und Antikörper gewonnen, die im Sinne der Erfindung verwendet werden können.

Ein solcher hochmolekularer Träger ist beispielsweise Albumin oder Keyhole Limpet Hämocyanin, woran das Peptid über eine Thioether-Bindung gekoppelt wird. Geeignete Koppelungstechniken sind auch solche, wobei die Peptide über Aminogruppen an ein Trägerprotein gebunden werden, beispielsweise die Glutardialdehydmethode.

Mit diesen Produkten als Immunisierungsantigenen können polyklonale oder monoklonale Antikörper hergestellt werden. Diese Antikörper können durch Affinitätschromatographie gereinigt werden. Als geeignet haben sich Affinitätsmaterialien erwiesen, die entweder ein wie oben beschriebenes Peptid als Liganden besitzen oder die Protein A zu diesem Zweck einsetzen.

Gegenstand der Erfindung sind auch solche Antikörper sowie ihre Verwendung als Arzneimittel.

| Abkürzungen | |
|---|---|
| Cys | L-Cystein |
| Ser | L-Serin |
| Thr | L-Threonin |
| Ala | L-Alanin |
| Val | L-Valin |
| Ile | L-Isoleucin |
| Arg | L-Arginin |
| Met | L-Methionin |
| Pro | L-Prolin |
| Glu | L-Glutaminsäure |
| Fmoc | 9-Fluorenylmethyloxycarbonyl |
| DMF | Dimethylformamid |
| tBu | tert.-Butyl |
| Mtr | 4-Methoxy-2,3,6-trimethylphenylsulfonyl |
| StBu | tert.-Butylmercapto |
| TFA | Trifluoressigsäure |
| EDTA | Ethylendiamintetraessigsäure |
| Nva | Norvalin |
| CHA | Cyclohexylalanin |
| Lys | L-Lysin |
| GMBS | gamma-Maleimidobuttersäure-N-hydroxysuccinimidester |

Die folgenden Beispiele erläutern die Erfindung näher.


Beispiel 1


a) Synthese von Cys-Ser-Thr-Ala-Val-Ile-Val-Ser-Ala-Arg-Met-Ala-Pro-Glu-Glu-Ile-Ile

Die Synthese wurde an einem halbautomatischen Peptidsynthesizer ausgeführt. 1 g Fmoc-Ile-p-alkoxybenzylesterpolystyrol (1 % Divinylbenzol quervernetzt) wurde mit 15 ml 20% (v/v) Piperidin/DMF behandelt und mit DMF und Isopropanol gewaschen. Es wurden 1,65 mmol Fmoc-Ile und 2,25 mmol HOBt gelöst in 14 ml DMF sowie 1,1 ml einer 1 M Diisopropylcarbodiimidlösung zugesetzt. Die Mischung wurde 1 Stunde bei Raumtemperatur geschüttelt und daraufhin mit DMF und Isopropanol so lange gewaschen, bis alle überschüssigen Reagenzien und löslichen Nebenprodukte entfernt waren. Dieses Verfahren wurde bis zum Erreichen der N-terminalen Aminosäure durchgeführt, wobei die folgenden Aminosäurenderivate benutzt wurden:
Fmoc-Glu (OtBu) Fmoc-Pro, Fmoc-Ala, Fmoc-Ile, Fmoc-Met, Fmoc-Arg (Mtr) Fmoc-Ser (tBu) Fmoc-Val, Fmoc-Thr (tBu), Boc-Cys(SStBu).
Das geschützte Peptidharz wurde mit 15 ml Trifluorethanol, 200 µl Wasser, 30 µl N-Methylmorpholin und 100 µl Butylphosphin versetzt und 12 Stunden bei Raumtemeraptur geschüttelt. Das Harz wurde mit 1 % (v/v) Eisessig in Methanol gewaschen und im Hochvakuum getrocknet. Das Harz wurde mit 18 ml TFA, 1 ml Thioanisol, 500 µl Dimercaptoethan und 500 mg Resorcin vermischt und 3 Stunden bei 35°C gerührt. Das abgespaltene Peptid wurde abfiltriert und mit 300 ml Diethylether kristallisiert. Das auskristallisierte Produkt wurde durch Gelpermeation an einer ®Sephadex-G25-Säule (3x100 cm, 0,5 % (v/v) Essigsäure) portionsweise (100 mg) gereinigt. Nach Lyophilisation wurden 328 mg Peptid gewonnen.


b) Konjugatherstellung

30 mg Keyhole Limpet Hämocyanin (KLH) wurden in 0,05 mmol/l Natriumphosphatpuffer pH 8,0 gelöst und mit 3 mg GMBS 1 Stunde lang gerührt. Das Protein wurde an einer ®Sephadex G 50-Säule (2x30 cm) (0,1 mol/l Natriumphosphat, 0,5 mmol/l EDTA pH 6,0) chromatographiert. Das aktivierte Protein wurde auf 5 ml konzentriert und mit 30 mg des Peptids 1 Stunde lang inkubiert. Nach Dialyse und Lyophilisation wurden 35 mg Immunisierungsantigen erhalten.

4

Beispiel 2

Immunisierung von Kaninchen

5 Kaninchen wurden mit jeweils 1,5 mg Antigen pro Tier über einen Zeitraum von 4 Wochen immunisiert. Das Peptid-KLH-Konjugat wurde subcutan und intravenös appliziert. Danach wurden die Tiere entblutet und die Rohantisera mit Konservierungsmittel stabilisiert. Ausbeute je Tier: 150 ml Antiserum.

Beispiel 3

Herstellung von Immunadsorbenzien

Die wie in Beispiel 2 dargestellt gewonnenen Rohantisera wurden affinitätschromatographisch gereinigt. Hierzu wurden 80 mg des 16er Peptides Ser-Thr-Ala-Val-Ile-Val-Ser-Ala-Arg-Met-Ala-Pro-Glu-Glu-Ile-Ile an 25 ml Bromcyan-aktivierte Sepharose nach dem von Axen et al. beschriebenen Verfahren (Nature 214, 1302, 1967) gekuppelt. Anschließend wurde das Immunadsorbens mit phosphatgepufferter Kochsalzlösung (PBS, 150 mmol/l, pH 7,2) und Essigsäure (500 mmol/l, pH 2,5) gewaschen und mit dem 3fachen Gelvolumen an PBS äquilibriert.

Beispiel 4

Gewinnung von Antikörpern

50 ml Rohantiserum passierten die mit PBS äquilibrierte Sepharose (gemäß Beispiel 3) mit einer Fließgeschwindigkeit von ca. 60 ml/h bei 23°C. Danach wurde mit dem dreifachen Säulenvolumen PBS, 1 M NaCl und aqua dest. (pH 7,0) gewaschen. Die Antikörper wurden eluiert mit Wasser (pH 2,5). Das Eluat wurde mit festem Tris-HCl auf pH 7,5 eingestellt und bei -20°C gelagert. Ausbeute: ca. 40 mg Antikörper.

Beispiel 5

Testung der immunadsortiv gewonnenen Antikörper

a) Blockierung der PAI-1-Aktivität im funktionellen Test

50 µl PAI-Mangelplasma, hergestellt durch Vorinkubation von 40 IU Urokinase/ml Plasma und anschließende Immunadsorption an Anti-Urokinase-Antikörper-Sepharose, und 50 µl PAI-reiches Plasma (10 Einheiten/ml) wurden mit 50 µl Antikörperlösung in PBS (gemäß Beispiel 4) sowie 1:10, 1:100 und 1:1000-Verdünnungen derselben, sowie nur PBS zur Kontrolle 15 min bei 23°C inkubiert und anschließend ein funktioneller Nachweis der PAI-Aktivität durchgeführt. Hierzu wurden 1 IU Urokinase in Tris-Puffer (100 mmol/l Tris, 100 mmol/l NaCl, 1 % Polygelin, 0,1 % (w/v) Triton X 100) zugefügt, 10 min bei 23°C inkubiert, 200 µl 10 mmol/l Chloramin T, 200 µl 10 CTA-U/ml Plasminogen in Trispuffer mit 10 mmol/l Tranexamsäure zugesetzt, für 10 min bei 23°C inkubiert. 500 µl 0,6 µmol/l chromogenes Plasminsubstrat Nva-CHA-Lys-pNA (pNA = para-nitroanilid) in 500 mmol/l NaCl starteten die Nachweisreaktion, die nach 10 min bei 23°C durch 100 µl 8,5 M Essigsäure abgestoppt wurde. Der Gehalt an funktionellem PAI einer Probe steht in linearer umgekehrt proportionaler Beziehung zur gemessenen Extinktionsänderung bei 405 nm.

Ergebnis:

EP 0 320 840 A2

|  | PAI-Mangelplasma (mE) | PAI-reiches Plasma (mE) |
|---|---|---|
| Zusatz: PBS | 800 | 350 |
| Antikörper 1:1000 | 805 | 368 |
| Antikörper 1:100 | 793 | 590 |
| Antikörper 1:10 | 810 | 685 |
| unverdünnt | 802 | 700 |

Ca. 80 % der Schnellinhibitionskapazität des PAI-reichen Plasmas konnten durch den Antikörper blockiert werden.

b) Immunadsorptive Entfernung von PAI-1-Aktivität aus biologischen Flüssigkeiten

14 mg Antikörper wurden an 10 ml Bromcyan-aktivierte Sepharose immobilisiert. 5 ml PAI-reiches Plasma wurden über die Sepharose in einer Fließgeschwindigkeit von 10 ml/h gegeben. Den Verdünnnungsfaktor berücksichtigend wurde eine Probe dieses Durchlaufs auf funktionelle PAI-1-Aktivität untersucht. Das Plasma hatte ca. 70 % seiner PAI-Aktivität verloren.

Beispiel 6

a) Synthese von Ala-Val-Ile-Val-Ser-Ala-Arg-Met-Ala-Pro-Glu-Cys

Ausgehend von 1 g Fmoc-Cys(SStBu)-p-alkoxybenzylester polystyrol wurde das Peptid in Analogie zu Beispiel 1 a) aufgebaut, abgespalten und gereinigt. Ausbeute 245 mg.

b) Konjugatherstellung

Die Konjugatherstellung erfolgte wie in Beispiel 1 b). Ausbeute 36 mg.

Immunisierung von Kaninchen, Herstellung von Immunadsorbens, Gewinnung der Antikörper erfolgte gemäß Beispiel 2 - 4 . Testung der immunadsorptiv gewonnenen Antikörper nach Beispiel 5 ergab PAI-Aktivität blockierende Antikörper (etwa 80 % der PAI-Aktivität blockiert) bis zur Titerstufe 1:20.

**Ansprüche**

1. Peptid enthaltend von 4 bis zu 40 Aminosäuren, dessen Sequenz mit der Aminosäuresequenz des Peptids Ser-Thr-Ala-Val-Ile-Val-Ser-Ala-Arg-Met-Ala-Pro-Glu-Glu-Ile-Ile oder mit Teilen dieser Sequenz übereinstimmt, und an das gegebenenfalls N- oder C-terminal Cystein gebunden ist, und das gegebenenfalls an einen Träger gebunden ist.

2. Peptid nach Anspruch 1, dadurch gekennzeichnet, daß es die Sequenz -Arg-Met- enthält.

3. Peptid nach Anspruch 1, dadurch gekennzeichnet, daß es 6 bis 25 Aminosäuren und die Sequenz Arg-Met enthält, wobei diese Sequenz von wenigstens 2 Aminosäuren flankiert ist.

4. Ser-Thr-Ala-Val-Ile-Val-Ser-Ala-Arg-Met-Ala-Pro-Glu-Glu-Ile-Ile.

5. Peptid nach Anspruch 1, dadurch gekennzeichnet, daß der N- oder C-Terminus Cystein ist.

6. Peptid nach Anspruch 1, wobei ein Terminus Cystein ist, gebunden an ein Polymer.

7. Cys-Ser-Thr-Ala-Val-Ile-Val-Ser-Ala-Arg-Met-Ala-ProGlu-Glu-Ile-Ile gebunden an ein Polymer.

8. Verfahren zur Herstellung eines Peptids nach Anspruch 1, dadurch gekennzeichnet, daß geschützte Aminosäurederivate oder Peptidsegmente in Lösung oder an einer Festphase aneinander gekoppelt werden und durch Abspaltung der Schutzgruppen sowie im Falle einer Festphase durch Abspaltung von Trägerharz Peptide gemäß Anspruch 1 erhalten werden.

9. Antikörper erhalten unter Verwendung eines gegebenenfalls an einen Träger gebundenen Peptid nach Anspruch 1 als Antigen.

6

10. Verwendung eines Antikörpers nach Anspruch 9 als Arzneimittel oder zur Diagnose.

Patentanspruch für folgende Vertragsstaaten: ES, GR

1. Verfahren zur Herstellung eines Peptids enthaltend von 4 bis zu 40 Aminosäuren, dessen Sequenz mit der Amino-säuresequenz des Peptids Ser-Thr-Ala-Val-Ile-Val-Ser-Ala-Arg-Met-Ala-Pro-Glu-Glu-Ile-Ile oder mit Teilen dieser Sequenz übereinstimmt, und an das gegebenenfalls N- oder C-terminal Cystein gebunden ist, dadurch gekennzeichnet, daß geschützte Aminosäurederivate oder Peptidsegmente in Lösung oder an einer Festphase aneinander gekoppelt werden und durch Abspaltung der Schutzgruppen sowie im Falle einer Festphase durch Abspaltung vom Trägerharz diese Peptide erhalten werden.